(19) Europäisches Patentamt
European Patent Office
Office européen des brevets

(11) EP 0 555 646 B1

(12) EUROPÄISCHE PATENTSCHRIFT

(45) Veröffentlichungstag und Bekanntmachung des Hinweises auf die Patenterteilung:
**24.06.1998 Patentblatt 1998/26**

(51) Int. Cl.⁶: **B07C 5/34**, G01N 21/90

(21) Anmeldenummer: **93100447.7**

(22) Anmeldetag: **14.01.1993**

(54) **Verfahren und Vorrichtung für die Schadstoffdetektion und -identifikation in Getränkeflaschen in Abfüllinien**

Method and device for detecting and identifying injurious substances in beverage bottles in a filling line

Procédé et dispositif de détection et d'identification de substances nocives dans les bouteilles de boisson d'une chaîne de remplissage

(84) Benannte Vertragsstaaten:
**AT BE CH DE DK ES FR GB GR IE IT LI NL PT SE**

(30) Priorität: **16.01.1992 DE 4200971**

(43) Veröffentlichungstag der Anmeldung:
**18.08.1993 Patentblatt 1993/33**

(73) Patentinhaber:
**Krieg, Gunther, Prof.Dr.Ing.
D-76227 Karlsruhe (DE)**

(72) Erfinder:
• **Koukolitschek, Karl Dipl.Ing.
W-7500 Karlsruhe 21 (DE)**
• **Krieg Gunther,Prof. Dr.Ing.
W-7500 Karlsruhe 41 (DE)**
• **Maier Wilfried
W-7519 Sulzfeld (DE)**

(74) Vertreter:
**Lempert, Jost, Dipl.-Phys. Dr. rer.nat.
Patentanwälte,
Dipl.-Ing. Heiner Lichti,
Dipl.-Phys. Dr. rer. nat. Jost Lempert,
Dipl.-Ing. Hartmut Lasch,
Postfach 41 07 60
76207 Karlsruhe (DE)**

(56) Entgegenhaltungen:
| | |
|---|---|
| **GB-A- 1 422 129** | **US-A- 3 601 616** |
| **US-A- 4 492 475** | **US-A- 4 551 627** |
| **US-A- 4 804 273** | **US-A- 4 858 768** |

• **TM TECHNISCHES MESSEN Bd. 58, Nr. 3 , März 1991 , MUNCHEN DE Seiten 122 - 127 XP000224812 STAAB 'INDUSTRIELLE GASANALYSE'**

## Beschreibung

Die Prüfung von Mehrwegflaschen und Mehrwegbehältern auf flüssige, feste und gasförmige Kontaminationen, ist ein wichtiges technisches Problem, insbesondere seit der Einführung von Kunststoffflaschen und Kunststoffbehältern, da die Giftstoffe in das Wandungsmaterial eindiffundieren und bei Wiederbefüllung mit dem jeweiligen Produkt, z.B. mit Limonade, mit Cola, mit Fruchtsäften etc., eine Geschmacksbeeinträchtigung verursachen.

Die in US-Patentschrift 4,858,768 angegebene Methode, die jeweilige, der Flasche entnommene Flüssigkeit dahingehend zu untersuchen, ob sie mit dem Orginalprodukt der Flasche bzw. des Behälters übereinstimmt und andernfalls die Flasche bzw. den Behälter aus dem Wiederbefüllungsprozess auszuscheiden, ist unwirtschaftlich, da Mehrwegflaschen/-Behälter sehr selten mit dem Orginalprodukt zur Abfüllfabrik zurückkehren. Demzufolge würde eine Vielzahl von Flaschen ungerechtfertigt ausgeschieden, da viele Gärprodukte, Spülung mit Wasser durch den Verbraucher, Austrocknen usw. zwar die Zusammensetzung des Orginalprodukts ändern, jedoch nicht dazu führen würden, daß die Flasche bzw. der Behälter als kontaminiert zu bezeichnen ist. Ferner führt die in der US-Patentschrift 4,858,768 beabsichtigte alleinige Untersuchung der in der Flasche/Behälter enthaltenen Restflüssigkeit ("residue") nicht zur Lösung des Problems, da die in der Flaschen- /Behälter- Wandung adsorbierten Schadstoffe nicht erfaßt würden.

US-A-4 551 627 und US-A-4 376 951 zeigen ein Verfahren und eine Vorrichtung zum Sortieren und Ausscheiden von Mehrwegbehältern nach dem Oberbegriff der Ansprüche 1 und 10.

Der Erfindung liegt daher die Aufgabe zugrunde, ein Verfahren und eine Vorrichtung zu schaffen, bei denen nicht nur auf dem Boden des Behälters abgesetzte Restflüssigkeiten, sondern auch Materialreste an oder in der Wandung des Behälters detektiert werden können. Erfindungsgemäß wird die genannte Aufgabe mit den Merkmalen der Ansprüche 1 und 10 gelöst.

Darüber hinaus gestattet eine bevorzugte Ausführungsform des Verfahrens auch eine Schadstoffanalyse von auf der Wand befindlichen Flüssigkeitsfilmen, wie z.B. Ölfilmen, Filmen von Reinigungsmitteln, von Waschmitteln etc., sowie eine Schadstoffanalyse des eingetrockneten Produkts, d.h. des Produktfilmes, und ferner von Feststoffilmen, wie z.B. dünnen Schichten von Farben, Lacken, Klebern, Wachsen, Kosmetikprodukten u.s.w.

Bevorzugt wird die zu untersuchende Flasche bzw. der Behälter mit elektromagnetischer Strahlung geeigneter Wellenlängenbereiche, die innerhalb des Gesamtintervalls zwischen den Wellenlängen von 200 nm bis 10 cm liegen, durchstrahlt. Die Wellenlängenbereiche werden so gewählt, daß eine hohe Durchlässigkeit der Flaschenwandung für die elektromagnetische Strahlung gewährleistet ist. Die Richtung der elektromagnetischen Strahlung wird erfindungsgemäß so eingestellt, daß sowohl der Flaschen-/Behälter-Boden, als auch die Flaschen-/Behälter-Wandung von der den Boden durchquerenden elektromagnetischen Strahlung durchsetzt wird. Aus der Wellenlängenabhängigkeit der Schwächung der elektromagnetischen Strahlung wird in einer bevorzugten Ausführungsform unter Berücksichtigung der elektromagnetischen Strahlungstransmission des Flaschen-/Behälter- Wandungsmaterials der jeweilige Schadstoff, der sich in der Restflüssigkeit und/ oder im Gasraum befindet, detektiert und on-line analysiert. Ferner wird simultan festgestellt, welches Produkt ursprünglich im Behälter bzw. in der Flasche abgefüllt war.

Die Erfindung wird im folgenden an Ausführungsbeispielen näher erläutert. Hierbei zeigen:

Fig.1: Den prinzipiellen Aufbau der Gesamtanordnung zur Schadstoffdetektion und Echtzeit-Schadstoffanalyse in der Restflüssigkeit, in der Flaschen-/Behälter-Wandung, in Flüssigkeits-/Feststoff-Filmen auf der Innenwandung.

Fig.2: Ergebnisse der Echtzeit-Schadstoffanalyse bzw. der Echtzeit-Rest- Produktanalyse in Mehrweg-Polyäthylen-Getränkeflaschen.

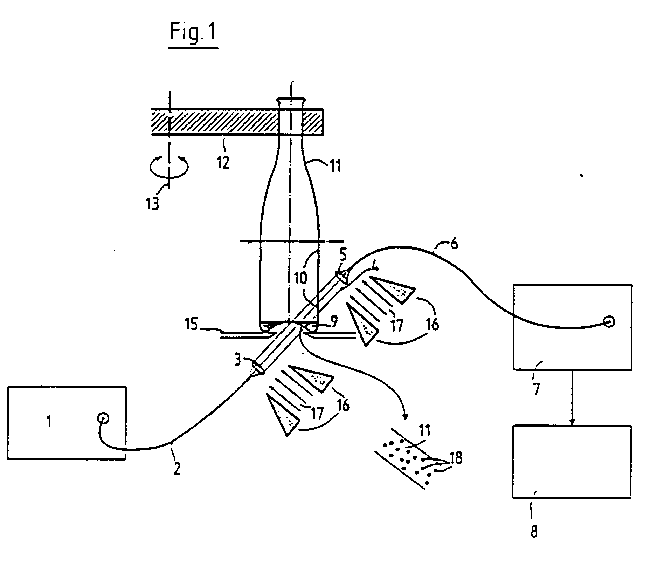

Gemäß Fig.1 wird die Flasche(11) / der Behälter(11) über eine Transportvorrichtung(12) in der Abfülllinie weiterbewegt. Die Transportvorrichtung kann gemäß Fig.1 eine Rotationsbewegung um die Achse(13) oder auch jede andere Bewegung, z.B. eine Linearbewegung, ausführen. Alternativ kann die Führungsfläche(15), die in der Ausführungsform von Fig.1 stationär ist, als Transportband zum Flaschen-/Behältertransport dienen.

Die Echtzeit-Stoffanalyse erfolgt durch ein on-line-Spektrometer(7) für elektromagnetische Strahlung mit nachgeschalteter Signalauswertung(8). Beide Einheiten(7,8) sind sowohl bezüglich Aufbau, als auch bezüglich ihrer Funktionen in DE-A-41 21 429 ausführlich beschrieben. Das on-line-Spektrometer(7) ist vorzugsweise für den ultravioletten-/sichtbaren- Bereich und/oder für den nahen, und/oder für den mittleren-Infrarotbereich des elektromagnetischen Spektrums ausgelegt. Eine Quelle(1) zur Erzeugung elektromagnetischer Strahlung übermittelt die Strahlungsenergie über ein Faserbündel(2) aus Quarzglas bzw. aus infrarottransparenten Fluor-Spezialverbindungen an die Linse(3). Letztere erzeugt einen annähernd parallelen Strahl(4), der die Flasche(11) in der Nähe des Champagnerbodens durchläuft und von einer Linse(5) auf ein zweites Faserbündel(6) fokussiert wird, um auf den Eingang des on-line-Spektrometers zu gelangen. Das Gesamtsystem gemäß Fig.1 erlaubt neben der Echtzeit-Analyse der in der Flasche(11) bzw. dem Behälter(11) enthaltenen Restflüssigkeit(9) insbesondere auch die Echtzeitanalyse von Flüssigkeits-/Festkörper-Filmen(10) an der Flascheninnenwandung. Desweiteren werden in der Flaschenwandung(11) infolge von Diffusionsprozessen eingelagerte Schadstoffmoleküle(18) identifiziert. Um möglichst viel Restflüssigkeit im Untersuchungsbereich zu erhalten, kann vorteilhafterweise vorgesehen sein, daß die Flasche 11 leicht bis maximal 45° gekippt wird. Dies kann durch entsprechende Ausbildung eines am Flaschenhals angreifenden Halters der Transportvorrichtung 12 geschehen.

Um Verschmutzungen im rauhen Betrieb weitgehend zu vermeiden. werden die notwendigerweise offen liegenden Teile(3,5) der Strahlführung über Düsen(16) mit Reinluft(17) gespült. Dadurch wird eine Kondensation von Schadstoffen, eine Ablagerung von Staubpartikeln, sowie eine Wasserdampf-Kondensation an den Fokussierungselementen (3,5) und anderen Komponenten verhindert.

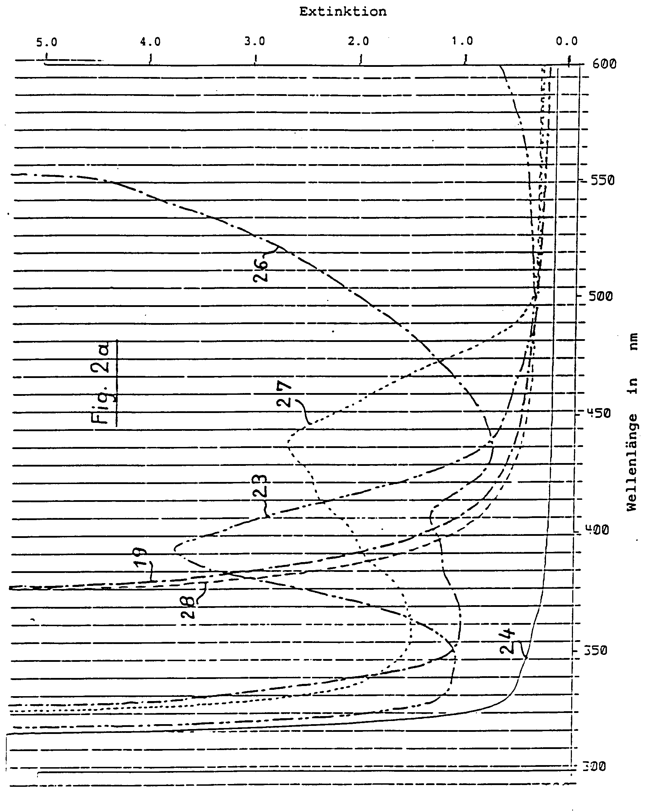

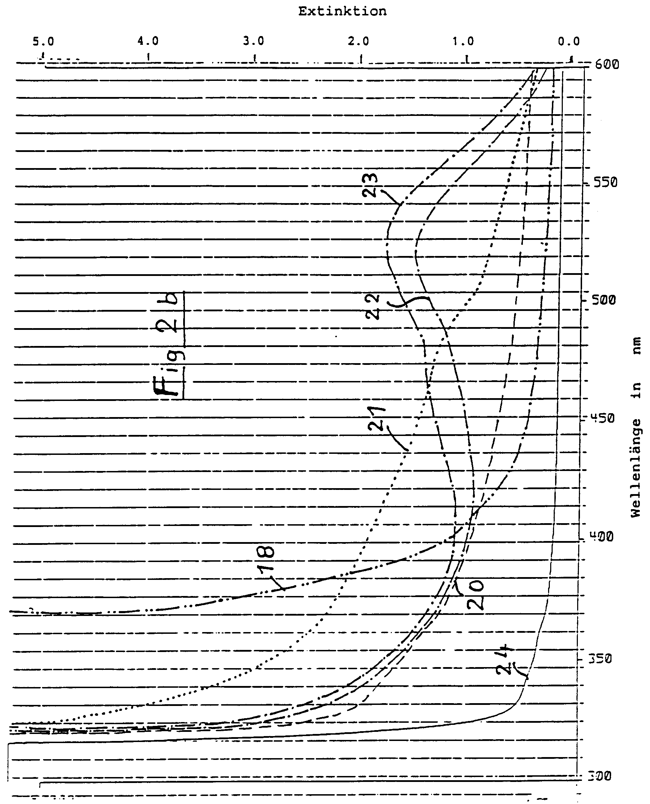

Ergebnisse der Echtzeit-Analyse unter Einsatz des in Fig.1 dargestellten Gesamtsystems zeigt Fig.2. In Fig.2a sind die Wellenlängenabhängigkeiten der logarithmischen Schwächung, der sogenannten Extinktion E, der elektromagnetischen Strahlung für eine neue, nicht kontaminierte Flasche(24), sowie für gefährliche, bzw. unerwünschte Schadstoff-Verunreinigungen der Restflüssigkeit(9), der Wandung(11) und von Schadstoff-Filmen(10) aufgetragen. Als Schadstoffe sind eine Nickelbad-Galvanik-Lösung(23), Tinte(26), Urin(19), Universalreiniger(27) sowie das Desinfektionsmittel NaClO (28), beispielhaft dargestellt. Die für jede Substanz typischen funktionalen Verläufe ermöglichen unter Einsatz der Signalauswertung (8) eine klare Detektion und Identifikation der Schadstoffe und insbesondere auch der in Fig. 2b entsprechend aufgeführten Funktionen für Lebensmittel, wie z.B. Cola(20) Erdbeersaft(22), Orangenlimonade(21), Pflanzenöl(18), Johannisbeersaft(23). Die für jede Substanz offensichtlich charakteristischen Funktionsverläufe ermöglichen eine selektive Ermittlung der Einzelsubstanzen, d.h. u.a. eine Unterscheidung zwischen giftigen, unerwünschten und erwünschten Substanzen und deren Konzentrationen, selbst wenn mehrere Giftstoffe bzw. Giftstoffe und Lebensmittel im Gemisch vorliegen. Werden Mineralwasserflaschen untersucht, so werden nur Flaschen als gut befunden, die ausschließlich Wasser als Restflüssigkeit enthalten; bei sämtlichen anderen Restflüssigkeiten, auch bei Säften, Limonaden od.dgl., erfolgt ein Aussortieren, da solche Inhalte auch nach einer Reinigung der Flaschen in wieder mit Mineralwasser befüllten Flaschen einen Nachgeschmack verursachen können.

Die diesbezügliche Auswertung bzw. Analyse erfolgt ausgehend von der in Fig.2 dargestellten Funktionen (18,19,20,21,22,23,24) durch mathematische Überlagerung dieser Funktion zu der vom System gemäß Fig.1 ermittelten jeweiligen Ergebnisfunktion. Dabei werden Methoden gemäß dem Stand der Technik eingesetzt, d.h. die Ergebnisfunktion E wird dargestellt als:

$$E(\lambda) = a_1 E_1(\lambda) + a_2 E_2(\lambda) + \ldots + a_n E_n(\lambda)$$

wobei $(\lambda)$ die Wellenlänge, $E_i(\lambda)$ der Funktionsverlauf für die Substanz i und $a_i$ Parameter sind, welche die Konzentrationswerte der jeweiligen Substanz i angeben.

**Patentansprüche**

1. Verfahren zum Sortieren und Ausscheiden von Mehrwegbehältern, insbesondere von Flaschen, in Abfüllinien, wobei elektromagnetische Strahlung die Böden der Behälter durchquert, dadurch gekennzeichnet, daß auch die Wandungen der Behälter von der die Böden durchquerenden elektromagnetischen Strahlung in der Nähe ihres Bodens durchsetzt werden, so daß Bereiche der Restflüssigkeit, Bereiche mit Flüssigkeitsfilmen, mit Festkörperfilmen an der Innenwand sowie Bereiche mit Kontaminationen innerhalb der Wandung von der elektromagnetischen Strahlung erfaßt werden können.

2. Verfahren nach Anspruch 1, dadurch gekennzeichnet, daß die elektromagnetische Strahlung einzelne Wellenlängenintervalle aufweist.

3. Verfahren nach Anspruch 1 bis 2 dadurch gekennzeichnet, daß die Wellenlängenintervalle insbesondere im ultravioletten-, sichtbaren-, nahen infrarot-, und im infrarot-Bereich liegen.

4. Verfahren nach Anspruch 1 bis 3, dadurch gekennzeichnet, daß die elektromagnetische Strahlung mit einem Echtzeit-Spektrometer zerlegt wird und daß die Wellenlängen-Abhängigkeit der Extinktion der Strahlung mit einer Signalauswertungseinheit berechnet wird.

5. Verfahren nach Anspruch 1 bis 4, dadurch gekennzeichnet, daß jeder Substanz eine Funktion der Extinktion in Abhängigkeit von der Wellenlänge zugeordnet wird.

6. Verfahren nach Anspruch 1 bis 5, dadurch gekennzeichnet, daß die Identifikation und Konzentrationsbestimmung der einzelnen Substanz durch Vergleich mit der zugeordneten Extinktionsfunktion erfolgt.

7. Verfahren nach Anspruch 1 bis 6, dadurch gekennzeichnet, daß die Identifikation und Konzentrationsbestimmung der Einzelsubstanzen eines Multikomponentengemisches durch Überlagerung der Extinktionsfunktionen der Einzelsubstanzen erfolgt.

8. Verfahren nach Anspruch 1 bis 7, dadurch gekennzeichnet, daß die elektromagnetische Strahlung über flexible Wellenleiter der Analyseeinrichtung zugeführt wird.

9. Verfahren nach Anspruch 1 bis 8, dadurch gekennzeichnet, daß die Komponenten zur Führung der elektromagnetischen Strahlen mit Luftdüsen unter Einsatz von Reinluft gespült werden.

10. Vorrichtung zum Sortieren und Ausscheiden von Mehrwegbehältern, insbesondere von Flaschen, in Abfüllinien, mit einer Einrichtung zum Durchstrahlen der Böden der Behälter mit elektromagnetischer Strahlung, dadurch gekennzeichnet, daß die Einrichtung auch zum Durchstrahlen der Wandungen der Behälter in der Nähe ihres Bodens eingestellt ist, so daß auch die Wandungen der Behälter von der die Böden durchquerenden Strahlung durchsetzt werden und so daß Bereiche der Restflüssigkeit(9), Bereiche mit Flüssigkeitsfilmen(10), mit Festkörperfilmen(10) an der Innenwand sowie Bereiche mit Kontaminationen(18) innerhalb der Wandung(11) von der elektromagnetischen Strahlung(4) erfaßbar sind.

11. Vorrichtung nach Anspruch 10, dadurch gekennzeichnet, daß die elektromagnetische Strahlung(4) einzelne Wellenlängenintervalle aufweist.

12. Vorrichtung nach Anspruch 10 bis 11 dadurch gekennzeichnet, daß die Wellenlängenintervalle insbesondere im ultravioletten-, sichtbaren-, nahen infrarot-, und im infrarot-Bereich liegen.

13. Vorrichtung nach Anspruch 10 bis 12, dadurch gekennzeichnet, daß die elektromagnetische Strahlung(4) mit einem Echtzeit-Spektrometer(7) zerlegt wird und daß die Wellenlängen-Abhängigkeit der Extinktion der Strahlung mit einer Signalauswertungseinheit(8) berechnet wird.

14. Vorrichtung nach Anspruch 10 bis 13, dadurch gekennzeichnet, daß jeder Substanz eine Funktion der Extinktion in Abhängigkeit von der Wellenlänge zugeordnet wird.

15. Vorrichtung nach Anspruch 10 bis 14 , dadurch gekennzeichnet, daß die Identifikation und Konzentrationsbestimmung der einzelnen Substanz durch Vergleich mit der zugeordneten Extinktionsfunktion erfogt.

16. Vorrichtung nach Anspruch 10 bis 15, dadurch gekennzeichnet, daß die Identifikation und Konzentrationsbestimmung der Einzelsubstanzen eines Multikomponentengemisches durch Überlagerung der Extinktionsfunktionen der Einzelsubstanzen erfolgt.

17. Vorrichtung nach Anspruch 10 bis 16, dadurch gekennzeichnet, daß die elektromagnetische Strahlung(4) über flexible Wellenleiter(2,6) der Analyseeinrichtung(7) zugeführt wird.

18. Vorrichtung nach Anspruch 10 bis 17, dadurch gekennzeichnet, daß die Komponenten zur Führung der elektromagnetischen Strahlen(3,5) mit Luftdüsen(16) unter Einsatz von Reinluft(17) gespült werden.

**Claims**

1. Method for sorting and removing disposable containers, particularly bottles, in filling lines, in which electromagnetic radiation traverses the bottoms of the containers, characterized in that also the walls of the containers are traversed by the electromagnetic radiation, which traverses the bottoms, in the vicinity of the base thereof, so that residual liquid areas, areas with liquid films, solid films on the inner wall and areas with contamination within the wall can be detected by the electromagnetic radiation.

2. Method according to claim 1, characterized in that the electromagnetic radiation has individual wavelength intervals.

3. Method according to claim 1 or 2, characterized in that the wavelength intervals are in particular in the ultraviolet, visible, near infrared and infrared ranges.

4. Method according to claims 1 to 3, characterized in that the electromagnetic radiation is dispersed with a real time spectrometer and that the wavelength dependence of the extinction of the radiation is calculated with a signal evaluating unit.

5. Method according to claims 1 to 4, characterized in that with each substance is associated a function of the extinction dependent on the wavelength.

6. Method according to claims 1 to 5, characterized in that the identification and concentration determination of the individual substances takes place by comparison with the associated extinction function.

7. Method according to claims 1 to 6, characterized in that the identification and concentration determination of the individual substances of a multicomponent mixture takes place by the superimposing of the extinction functions of the individual substances.

**8.** Method according to claims 1 to 7, characterized in that the electromagnetic radiation is supplied by means of flexible waveguides to the analytical device.

**9.** Method according to claims 1 to 8, characterized in that the components for guiding the electromagnetic beams are scavenged with air nozzles using pure air.

**10.** Apparatus for sorting and removing disposable containers, particularly bottles, in filling lines, with a device for transilluminating the bottoms of the containers with electromagnetic radiation, characterized in that the device is also set up for transilluminating the walls of the containers in the vicinity of the base thereof, so that also the walls of containers are traversed by the radiation traversing the bottoms and in this way areas of residual liquid (9), areas with liquid films (10), with solid films (10) on the inner wall and areas with contamination (18) within the wall (11) are detectable by the electromagnetic radiation (4).

**11.** Apparatus according to claim 10, characterized in that the electromagnetic radiation (4) has individual wavelength intervals.

**12.** Apparatus according to claim 10 or 11, characterized in that the wavelength intervals are particularly in the ultraviolet, visible, near infrared and infrared ranges.

**13.** Apparatus according to claims 10 to 12, characterized in that the electromagnetic radiation is dispersed with a real time spectrometer (7) and that the wavelength dependence of the extinction of the radiation is calculated with a signal evaluating unit (8).

**14.** Apparatus according to claims 10 to 13, characterized in that with each substance is associated a function of the extinction dependent on the wavelength.

**15.** Apparatus according to claims 10 to 14, characterized in that the identification and concentration determination of the individual substances takes place by comparison with the associated extinction function.

**16.** Apparatus according to claims 10 to 15, characterized in that the identification and concentration determination of the individual substances of a multicomponent mixture takes place by superimposing the extinction functions of the individual substances.

**17.** Apparatus according to claims 10 to 16, characterized in that the electromagnetic radiation (4) is supplied by means of flexible waveguides (2, 6) to the analytical device (7).

**18.** Apparatus according to claims 10 to 17, characterized in that the components for guiding the electromagnetic beams (3, 5) are scavenged with air nozzles (16) using pure air (17).

**Revendications**

1. Procédé de tri et de rejet de récipients consignés, en particulier de bouteilles dans les chaînes de remplissage, dans lequel les fonds des récipients sont traversés par un rayonnement électromagnétique, caractérisé en ce que les parois au niveau du fond des récipients sont également traversées par le rayonnement électromagnétique traversant les fonds de telle sorte que les zones contenant les restes du liquide, les zones de la paroi intérieure recouvertes de films de liquides ou de particules solides ainsi que les zones présentant des contaminations à l'intérieur de la paroi puissent être détectées par le rayonnement électromagnétique.

2. Procédé selon la revendication 1 caractérisé en ce que le rayonnement électromagnétique est présent sur des plages individuelles de longueurs d'onde.

3. Procédé selon les revendications 1 à 2 caractérisé en ce que les plages de longueur d'onde se situent en particulier dans le domaine des ultraviolets, visibles, proches des infrarouges et dans le domaine des infrarouges.

4. Procédé selon les revendications 1 à 3, caractérisé en ce que le rayonnement électromagnétique est décomposé par un spectromètre en temps réel et en ce que l'on calcule la dépendance par rapport aux longueurs d'onde de l'extinction du rayonnement au moyen d'une unité d'analyse des signaux.

5. Procédé selon les revendications 1 à 4, caractérisé en ce que l'on attribue à chaque substance une fonction d'extinction dépendant de la longueur d'onde.

6. Procédé selon les revendications 1 à 5, caractérisé en ce que l'on identifie individuellement chaque substance et que l'on détermine sa densité en la comparant à la fonction d'extinction correspondante.

7. Procédé selon les revendications 1 à 6, caractérisé en ce que l'on identifie chaque substance individuelle d'un mélange à composants multiples et que l'on détermine sa densité en superposant les fonctions d'extinction des différentes substances.

8. Procédé selon les revendications 1 à 7, caractérisé en ce que l'on conduit le rayonnement électromagnétique au dispositif d'analyse au moyen d'un guide d'ondes flexible.

9. Procédé selon les revendications 1 à 8, caractérisé en ce que les composants servant à guider les rayons électromagnétiques sont lavés par de l'air purifié sortant de buses d'air.

10. Dispositif de tri et de rejet des récipients consignés, en particulier des bouteilles dans les chaînes de remplissage comportant un dispositif pour examiner par rayonnement électromagnétique les fonds des récipients, caractérisé en ce que le dispositif est réglé de manière à pouvoir également examiner par rayonnement les parois des récipients au niveau de leur fond de sorte que les parois des récipients sont également traversées par les mêmes rayons qui traversent les fonds des récipients et qu'ainsi les zones contenant les restes de liquides (9), les parties recouvertes de films de liquides (10), de particules solides (10) sur la paroi intérieure ainsi que les zones comportant des contaminations (18) à l'intérieur de la paroi (11) puissent être détectées par le rayonnement (4) électromagnétique.

11. Dispositif selon la revendication 10 caractérisé en ce que le rayonnement électromagnétique (4) est présent sur des plages individuelles de longueurs d'onde.

12. Dispositif selon les revendications 10 à 11, caractérisé en ce que les plages de longueur d'onde se situent en particulier dans le domaine des ultraviolets, visibles, proches des infrarouges et dans le domaine des infrarouges.

13. Dispositif selon les revendications 10 à 12, caractérisé en ce que le rayonnement électromagnétique (4) est décomposé par un spectromètre en temps réel (17) et en ce que l'on calcule la dépendance par rapport à la longueur d'onde de l'extinction du rayonnement au moyen d'une unité d'analyse (8) des signaux.

14. Dispositif selon les revendications 10 à 13, caractérisé en ce que l'on attribue à chaque substance une fonction d'extinction dépendante de la longueur d'onde.

15. Dispositif selon les revendications 10 à 14, caractérisé en ce que l'on identifie chaque substance individuelle et que l'on détermine sa densité en la comparant à la fonction d'extinction correspondante.

16. Dispositif selon les revendications 10 à 15, caractérisé en ce que l'on identifie chaque substance individuelle d'un mélange à composants multiples et que l'on détermine sa densité en superposant les fonctions d'extinctions des différentes substances.

17. Dispositif selon les revendications 10 à 16, caractérisé en ce que l'on conduit le rayonnement électromagnétique (4) au dispositif d'analyse (7) au moyen de guides d'ondes flexibles (2,6).

18. Dispositif selon les revendications 10 à 17, caractérisé en ce que les composants servant à guider les rayons électromagnétiques (3,5) sont épurés par l'air purifié (17) envoyé par des buses d'air (16).

Fig.1

Fig. 2a

Extinktion

Wellenlänge in nm

Fig 2 b